# EUROPEAN PATENT SPECIFICATION

(11) **EP 4 189 120 B1**
(45) Date of publication and mention of the grant of the patent: **21.10.2026**
(21) Application number: 21759417.5
(22) Date of filing: 30.07.2021
(51) Int. Cl.: C12Q 1/6886

(54) **METHOD FOR IN VITRO DIAGNOSIS OF THE RISK OF RECURRENCE OR PERSISTENCE OF HEAD AND NECK CANCER (HNSCC) AND ASSOCIATED KIT**
VERFAHREN ZUR IN-VITRO-DIAGNOSE DES RISIKOS DES WIEDERAUFTRETENS ODER DER PERSISTENZ VON KOPF-HALS-KREBS (HNSCC) UND ZUGEHÖRIGES KIT
MÉTHODE DE DIAGNOSTIC IN VITRO DU RISQUE DE RÉCIDIVE OU DE PERSISTANCE DU CANCER DE LA TÊTE ET DU COU (HNSCC) ET KIT ASSOCIÉ

(30) Priority: 03.08.2020 IT 202000019024
(43) Date of publication of application: 07.06.2023
(73) Proprietor: Istituti Fisioterapici Ospitalieri, 00144 Roma (IT)
(72) Inventor: GANCI, Federica, 00144 Roma (RM) (IT); MANCIOCCO, Valentina, 00144 Roma (RM) (IT); PELLINI, Raul, 00144 Roma (RM) (IT); BLANDINO, Giovanni, 00144 Roma (RM) (IT)
(74) Representative: Gitto, Serena
(86) International application number: PCT/IT2021/050237
(87) International publication number: WO 2022/029811

(56) References cited:
- WO-A1-2018/032062
- US-A1- 2011 143 950
- WENYING YAN ET AL: "Comparison of Prognostic MicroRNA Biomarkers in Blood and Tissues for Gastric Cancer", JOURNAL OF CANCER, vol. 7, no. 1, 1 January 2016 (2016-01-01), AU, pages 95 - 106, XP055580850, ISSN: 1837-9664, DOI: 10.7150/jca.13340
- PEDERSEN NICKLAS JUEL ET AL: "MicroRNA-based classifiers for diagnosis of oral cavity squamous cell carcinoma in tissue and plasma", ORAL ONCOLOGY, vol. 83, 1 August 2018 (2018-08-01), GB, pages 46 - 52, XP055831875, ISSN: 1368-8375, DOI: 10.1016/j.oraloncology.2018.05.020
- FUJIMOTO SHUNTARO ET AL: "Distinct spectrum of microRNA expression in forensically relevant body fluids and probabilistic discriminant approach", SCIENTIFIC REPORTS, vol. 9, no. 1, 4 October 2019 (2019-10-04), US, XP093404659, ISSN: 2045-2322, DOI: 10.1038/s41598-019-50796-8
- GANCI FEDERICA ET AL: "Altered peritumoral microRNA expression predicts head and neck cancer patients with a high risk of recurrence", vol. 30, no. 10, 21 July 2017 (2017-07-21), GB, pages 1387 - 1401, XP055796314, ISSN: 0893-3952, Retrieved from the Internet <URL:http://www.nature.com/articles/modpathol201762> DOI: 10.1038/modpathol.2017.62
- VAHABI MAHROU ET AL: "miR-96-5p targets PTEN expression affecting radio-chemosensitivity of HNSCC cells", vol. 38, no. 1, 29 March 2019 (2019-03-29), XP055796340, Retrieved from the Internet <URL:http://link.springer.com/article/10.1186/s13046-019-1119-x/fulltext.html> DOI: 10.1186/s13046-019-1119-x
- HARADA KAZUTO ET AL: "The role of microRNA in esophageal squamous cell carcinoma", JOURNAL OF GASTROENTERLOGY, SPRINGER JAPAN KK, JP, vol. 51, no. 6, 21 January 2016 (2016-01-21), pages 520 - 530, XP035885919, ISSN: 0944-1174, [retrieved on 20160121], DOI: 10.1007/S00535-016-1161-9

## Description

The present invention relates to a method for *in vitro* diagnosis of the risk of recurrence or persistence of head and neck cancer (HNSCC) and associated kit. In particular, the present invention relates to a method for *in vitro* diagnosis of the risk of recurrence or persistence of head and neck cancer (HNSCC) and an associated kit, wherein the diagnosis of the degree of risk of recurrence or persistence of the cancer is performed by microRNA detection in liquid biopsy samples, in particular in serum and saliva samples.

It is well known that head and neck cancers (HNSCC) originate from the oral cavity, larynx and pharynx and represent the sixth most frequent type of neoplastic pathology in the world, with about 500,000 new cases per year, and one of the lowest five-year rates of survival after diagnosis. At present, the main therapeutic strategy consists in surgical treatment of the cancerous lesion followed, based on the type of tumour, by adjuvant radio- and/or chemoradiotherapy.

The development of local recurrence, which occurs in over 50% of cases, is the main cause of death in patients affected by this type of cancer and the diagnosis thereof is made difficult by the transformations that the tissues subjected to radiotherapy undergo during treatment.

At present, the prognosis and the oncologist's decision as to which therapeutic strategy should be applied to each patient is mainly tied to the TNM staging of the tumour. However, the TNM classification system, based on an assessment of the histological examination, is limited in its ability to predict the patient's prognosis, as it does not sufficiently reflect the molecular heterogeneity of the tumour. Therefore, patients classified with the same TNM stage can have a completely different therapeutic response and clinical course.

Furthermore, the high rate of mortality observed in the event of recurrence is tied in part to the ineffectiveness of the currently used methods of investigation (histology and imaging) in making an early, clear determination of the recurrence of the tumour, thus decreasing the possibility of success of the treatment of the patient. This difficulty is often tied to the presence of tissue lesions caused by radiotherapy (which most HNSCC patients undergo) and which can be confused with cancerous lesions, making the diagnosis of recurrence uncertain and delaying the possible beginning of a treatment.

A further disadvantage of the diagnostic methods currently used is the invasiveness characterising the collection of a histological sample.

Therefore, the complete absence of diagnostic tests that can be performed in association with histology and imaging in the clinical course of a patient is a major problem.

In the light of the foregoing, there appears to be an evident need to provide new diagnostic methods for identifying the occurrence of a relapse or persistence of HNSCC tumours which overcome the disadvantages of the known methods of diagnosis.

Experimental evidence shows that microRNAs, small RNAs with a length of 22 nucleotides capable of binding mRNAs and blocking their production, show an altered expression in different types of cancer, in a tissue-specific manner, including in head and neck cancers (HNSCC) (Heneghan et al., 2010; Ramdas et al., 2009).

More recently, their potentialities not only as tissue molecular markers, but also as circulating biomarkers detectable in various types of biological fluids have been highlighted (Szelenberger et al., 2019, Cui et al., 2019). This potential derives from the ability of cells to secrete miRNAs in a tissue-specific manner and their extreme stability, also outside the cell.

However, not all miRNAs have diagnostic and/or prognostic value and not all deregulated miRNAs in tumour tissues, compared to a counterpart that is histologically negative for the presence of tumour cells, are also detectable in a liquid biopsy (Pedersen et al., 2018). Furthermore, some miRNAs can have diagnostic/prognostic value if detected in tissue, but not have diagnostic/prognostic value in a liquid biopsy, despite being detectable, or they can be correlated to different clinical parameters depending on whether they are detected in tissue or in circulation (Yan et al., 2016). Finally, depending on the type of liquid biopsy sample under analysis (saliva or blood), different miRNAs can be detected. For example, a miRNA can be detected and correlated to the prognosis or diagnosis of patients if its expression is evaluated in blood and can lose this correlation with the prognosis or diagnosis if detected in saliva samples or vice-versa (Fujimoto et al., 2019)

In particular, in head and neck cancers there are two known studies (Ganci et al., 2013; Ganci et al., 2017) concerning the levels of expression of several miRNAs in tissues from 132 HNSCC patients belonging to the cohort of the IRCCS Italian National Cancer Institute "Regina Elena" (IRE).

In the first study (Ganci et al., 2013) a 12 miRNA signature was identified, whose expression in tumour tissue was correlated with the development of recurrence. In particular, high levels of the signature are indicative of a higher probability of development of local recurrence. A second signature of 4 miRNAs (3 of which also included in the first signature), correlated with patient survival, was then identified in the study. Finally, it was shown in the study that the 12 miRNAs also have a role individually as independent prognostic factors. The second study (Ganci et al., 2017) focused instead on identifying miRNAs whose alteration is already visible in a histologically healthy peritumoral tissue. In particular, the paper identifies 35 already deregulated microRNAs in peritumoral tissue as compared to the surgical resection margin, 4 of which having prognostic value. In this case as well, the miRNAs work as prognostic biomarkers both individually and as signatures.

Summing up, the two above-mentioned studies show that high levels of expression of certain miRNAs, including miR-21-5p, miR-21-3p, miR-96-5p and miR-429, in tumoral and peritumoral tissues are correlated with a higher probability of recurrence developing in HNSCC patients. Furthermore, in the aforesaid studies it is shown that high levels of miR-21-5p and miR-21-3p are also correlated with a lower probability of patient survival.

The solution according to the present invention fits into this context, aiming to provide a new method of *in vitro* diagnosis for detecting the degree of risk of recurrence of HNSCC in patients undergoing tumour removal, for example by surgical treatment of the cancerous lesion.

The invention is defined by the claims. Any subject-matter falling outside the scope of the claims is provided for information purposes only. According to the present invention, it has now been surprisingly found that the microRNAs miR-21-3p, miR-96-5p and miR-429 can be detected in liquid biopsy samples of patients affected by head and neck cancer; serum and saliva, in particular, are strongly correlated with the development of recurrence or persistence of the cancer, specifically in HNSCC in patients undergoing tumour removal, for example by means of surgical treatment.

According to the present invention, a method for *in vitro* diagnosis of the risk of recurrence or persistence of HNSCC has thus been developed.

The method according to the present invention consists of a simple, non-invasive molecular test based on measurement of the expression and evaluation of two or all of the circulating miRNAs miR-21-3p, miR-96-5p and miR-429, and optionally also of the microRNA miR-21-5p; or of one of the miRNAs selected from miR-21-3p, miR-96-5p and miR-429 and also of the microRNA miR-21-5p, in liquid biopsy samples, in particular in serum and saliva, before and after removal of the tumour, for the diagnosis of the degree of risk of recurrence or persistence of the HNSCC. In particular, molecular analyses of saliva samples could be more informative for cancers of the oral cavity, whereas those performed on serum could be more suitable for the other two head and neck cancer sites (pharynx and larynx) (Wang et al., 2015). The method according to the invention, moreover, can further comprise measurement of the miRNA miR-21-5p.

MiRNAs are extremely stable molecules. The fact of detecting the aforesaid miRNAs in liquid biopsy samples, in particular in saliva, makes the method according to the invention a method that is not invasive for the patient, who is already stressed by treatments and by the disease, unlike the known methods that entail collecting tissue samples by biopsy. Consequently, the method according to the invention can be advantageously used every time it is deemed necessary for follow-up purposes and to monitor the patient's response to therapy. Furthermore, the result is obtained in a few hours and the technology preferably used (TaqMan assay) is highly specific and sensitive.

The method according to the present invention, therefore, advantageously makes it possible to:
- define the molecular nature of the tumour and, consequently, choose the therapeutic strategy that may be more effective for the patient;
- provide a non-invasive method for monitoring and follow-up, as well as for assessing the response to therapy, if any;
- monitor the patient's clinical course, thus enabling an early detection of any onset of recurrence or persistence of the HNSCC tumour and increasing the probability of patient survival;
- confirm or not confirm the presence of a suspected recurrence, in particular in cases in which the tissue lesion found is of dubious nature.

As highlighted above, the methods of investigation currently used in the treatment of HNSCC patients are very limited in terms of their diagnostic and prognostic capacity. The introduction of the molecular test according to the method of the present invention can advantageously provide an important aid to clinicians in formulating a patient diagnosis and prognosis that is as realistic as possible.

The method according to the invention comprises detecting two, three or all of the miRNAs among the ones mentioned above. The fact of using the three miRNAs (or four miRNAs, when miR-21-5p is also detected) as biomarker signatures is important, because the combination of the above-mentioned miRNAs increases the prognostic and diagnostic power of the method. However, in the event that one or more miRNAs are not reliably detectable in some samples or are not expressed in a given sample at a given time, the prognostic power of the remaining miRNAs will not be lost and will remain in any case reliable.

As stated above, the method according to the present invention can be advantageously used to diagnose patients with a high risk of recurrence through the detection of the above-mentioned miRNAs within liquid biopsies such as serum and saliva taken from patients whose diagnosis of HNSCC is already histologically confirmed. In particular, according to the present invention, a diagnostic protocol has been developed which offers the advantage of overcoming the problem of variability of expression which may occur between one person and another and may not be strictly cancer-associated.

Furthermore, in cases of patients undergoing radiotherapy and an uncertain diagnosis of recurrence, the method according to the present invention can be advantageously used to confirm or not confirm the suspicion of disease relapse or persistence.

As shown by the experimental data provided further below, the miRNAs miR-21-3p, miR-96-5p and miR-429 have surprisingly proven to be detectable in liquid biopsy samples. It has further been confirmed that miR-21-5p is also detectable in liquid biopsy samples, such as blood and saliva.

Furthermore, according to the present invention, the prognostic value of the above-mentioned miRNAs in the diagnosis of HNSCC tumour relapse has been shown. In particular, it has been observed that in non-relapsing patients the expression of the miRNAs according to the present invention decreases over time after tumour removal compared to the expression of the same miRNAs before tumour removal.

Therefore, on the basis of the results obtained, it can be affirmed that it is possible to correlate the expression of the miRNAs miR-21-3p, miR-96-5p and miR-429 and, optionally, also of miR-21-5p, in liquid biopsy samples, with the patient's clinical course.

In particular, the presence of an increased or unchanged expression of two, three or all of the above-mentioned miRNAs in liquid biopsy samples taken at times after the surgical removal of the tumour, compared to the expression thereof detected in the preoperative condition (thus in the presence of the tumour), thus serves to indicate a return of disease or residual disease.

It is therefore a specific object of the present invention a method for *in vitro* diagnosis of the degree of risk of recurrence (i.e. the degree of risk of having developed recurrence or of developing a recurrence) or risk of persistence of head and neck cancer in a patient who has undergone removal of the head and neck tumour, for example by surgical treatment, said method comprising or consisting of measuring, in at least one liquid biopsy sample collected before tumour removal and in at least one liquid biopsy sample collected after tumour removal, the expression of two or all of the miRNAs selected from miR-21-3p, miR-96-5p and miR-429, optionally also of the microRNA miR-21-5p; or
of one of the miRNAs selected from miR-21-3p, miR-96-5p and miR-429 and also of the microRNA miR-21-5p, wherein an expression of said miRNAs in said at least one liquid biopsy sample collected after tumour removal that is equal to or greater than the expression of said miRNAs in said at least one liquid biopsy sample collected before tumour removal indicates a high risk of recurrence or persistence of the cancer, whereas a lower expression indicates a low risk of recurrence or persistence of the cancer.

Therefore, the method according to the present invention uses the following combinations of microRNAs as biomarkers:
miR-21-3p and miR-96-5p, optionally also miR-21-5p;
miR-21-3p and miR-429, and optionally also miR-21-5p;
miR-96-5p and miR-429, optionally also miR-21-5p;
miR-21-3p, miR-96-5p and miR-429, optionally also miR-21-5p; or
one of the miRNAs miR-21-3p, miR-96-5p and miR-429 in combination with miR-21-5p.

In the context of the present invention, one may speak of recurrence when, six months after the initial treatment, the reappearance of the head and neck cancer has been diagnosed in the same site as the primary tumour and/or in one of the lymph nodes situated in the neck region.

Instead, if such events are diagnosed within six months after the initial treatment, they are considered persistence of the cancer or persistence of disease. Furthermore, it has been shown that miRNA 96-5p has a role in indicating the response to radio- and/or chemotherapy in HNSCC cells (Vahabi et al., 2019); this power is maintained in samples of serum, but not of saliva. In particular, after adjuvant treatment, high levels of this miRNA in serum compared to the preoperative sample can indicate a higher probability of having developed resistance to the therapeutic treatment underway and thus, consequently, a higher probability of developing recurrence. This data can therefore provide an important indication as to the possibility of modifying the therapeutic strategy for a given patient.

Therefore, according to one embodiment of the method according to the present invention, said two miRNAs or said one of the miRNAs in combination with miR-21-5p comprise miR-96-5p when the subject undergoes radio- and/or chemotherapy, in order to verify the effectiveness of the treatment or the development of resistance to the treatment. In such a case, the miR-96-5p is detected in a serum, plasma or blood sample.

According to the present invention, said at least one liquid biopsy sample can be selected from blood, plasma, serum and saliva.

Again according to the present invention, the expression of miR-96-5p can be measured in a blood, serum or plasma sample, the expression of miR-429 can be measured in a saliva sample and the expression of miR-21-3p or of miR-21-5p can be measured in blood, plasma, serum or saliva.

Preferably, the method according to the present invention is carried out on one or more samples selected from blood, serum or plasma and on a saliva sample. In fact, according to the method of the present invention, the expression of the miRNAs can also be measured in multiple liquid biopsy samples of a different type among the ones mentioned above, such as, for example, in a plasma or serum sample and in a saliva sample.

The method according to the present invention can be carried out by Real Time PCR, Microarray or RNA Next Generation Sequencing.

The subject matter of the present invention further relates to the use of two or all of the microRNAs selected from miR-21-3p, miR-96-5p and miR-429, optionally in combination with microRNA miR-21-5p; or
one of the miRNAs selected from miR-21-3p, miR-96-5p and miR-429 in combination with the microRNA miR-21-5p, as biomarkers for the *in vitro* diagnosis of the degree of risk of recurrence or persistence of the head and neck cancer in liquid biopsy samples.

Therefore, the use according to the present invention employs the following combinations of microRNAs as biomarkers:
miR-21-3p and miR-96-5p, optionally also miR-21-5p;
miR-21-3p and miR-429, and optionally also miR-21-5p;
miR-96-5p and miR-429, optionally also miR-21-5p;
miR-21-3p, miR-96-5p and miR-429, optionally also miR-21-5p; or
one of the miRNAs miR-21-3p, miR-96-5p and miR-429 in combination with miR-21-5p.

As stated above, the liquid biopsy samples can be selected from blood, plasma, serum and/or saliva.

The present invention also relates to a method of treating head and neck cancer in a subject, said method comprising
measuring, in at least one liquid biopsy sample taken before tumour removal and in at least one liquid biopsy sample taken after tumour removal, the expression of two or all of the miRNAs selected from miR-21-3p, miR-96-5p and miR-429, optionally in combination with the microRNA miR-21-5p; or
of one of the miRNAs selected from miR-21-3p, miR-96-5p and miR-429 in combination with the microRNA miR-21-5p,
and, when the expression of said miRNAs in said at least one liquid biopsy sample taken after tumour removal is equal to or greater than the expression of said miRNAs in said at least one liquid biopsy sample taken before tumour removal,administering to the subject a therapy against head and neck cancer, such as, for example, radio- and/or chemotherapy. For example, drugs inhibiting the PI3K/Akt/mTOR pathway can be administered.

Therefore, the method according to the present invention uses the following combinations of microRNAs as biomarkers:
miR-21-3p and miR-96-5p, optionally also miR-21-5p;
miR-21-3p and miR-429, and optionally also miR-21-5p;
miR-96-5p and miR-429, optionally also miR-21-5p;
miR-21-3p, miR-96-5p and miR-429, optionally also miR-21-5p; or
one of the miRNAs miR-21-3p, miR-96-5p and miR-429 in combination with miR-21-5p.

The subject matter of the present invention further relates to a kit for *in vitro* diagnosis of the degree of risk of recurrence or persistence of head and neck cancer, said kit comprising or consisting of one or more pairs of primers and/or one or more probes for the detection of two or all of the microRNAs selected from miR-21-3p, miR-96-5p and miR-429, optionally also for the detection of the microRNA miR-21-5p; or
of one of the miRNAs selected from miR-21-3p, miR-96-5p and miR-429 in combination with primers and/or probes for the detection of the microRNA miR-21-5p.

According to a particular embodiment of the method according to the present invention, several samples are collected at different times, according to the following protocol:
- sample A) taken before the surgical treatment;
- sample B) taken the day after the intervention;
- sample C) taken 15 days after the surgical intervention;
- subsequent samples D), E), F) and so on, taken during the follow-up visits planned for the patient.

The biological samples thus collected can be processed within 1 hour of being collected and stored at -80°C pending further processing. For the extraction of RNA, one waits to collect at least samples B and C. After collecting at least these first samples, one proceeds with the RNA extraction and evaluation of the expression of the miRNAs of the invention both in saliva and in serum.

As the expression of the miRNAs can vary greatly from patient to patient, in order to evaluate the pattern of expression thereof by analysing the collected samples, the method according to the invention advantageously envisages that, for each patient, the expression of the miRNAs in the same patient in the presence of a tumour (sample A) will be considered the reference level.

The modulation of the miRNAs in the subsequent samples is then calculated, considering them both together as signatures and individually. Based on the type of modulation observed in the samples following the baseline sample, the patient is classified as at low risk (if the expression is lowered) or high risk of recurrence (if the expression remains equal or rises). The samples taken during the follow-up will thus be evaluated on the basis of sample A, together with histological analyses and imaging tests (if provided for). The modulation of the miRNAs found at this stage will be indicative of the patient's clinical course, of his or her response to therapy and any onset of a relapse. Using the value of expression of the miRNAs detected in the patient's sample A (condition in the presence of the tumour) offers the advantage of overcoming the problem of variability of expression that may exist between one person and another and may not be strictly cancer-associated.

The present invention will now be described by an illustrative, but non-limiting, way according to a preferred embodiment thereof, with particular reference to the examples and attached figures, wherein:

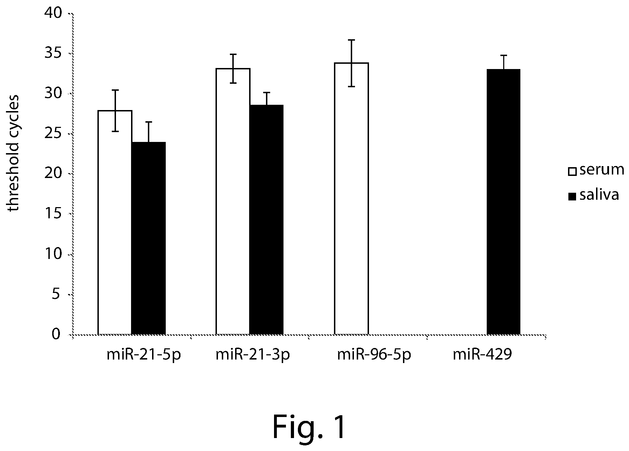

- **Figure 1** shows that a measurement of miR-21-3p, miR-96-5p, miR-429 and miR-21-5p can be made in the two types of liquid biopsy (serum and saliva) coming from patients with HNSCC. In particular, the graph represents the non-normalised raw data corresponding to the RT-qPCR threshold cycles for each miR in the two types of liquid biopsy on which the test is based. For each miR, the mean of the cycles calculated for 8 serum samples and 4 saliva samples taken at the time the tumour was present (preoperative sample A) is shown. The threshold cycles are inversely correlated to the expression of the miR: the higher the cycle, the less the miR will be expressed. Threshold cycles <29 are indicative of a strong expression of the miR, whilst threshold cycles comprised between 30-37 are indicative of a moderate expression of the miR; finally, above 37 cycles the miR is not considered as expressed;

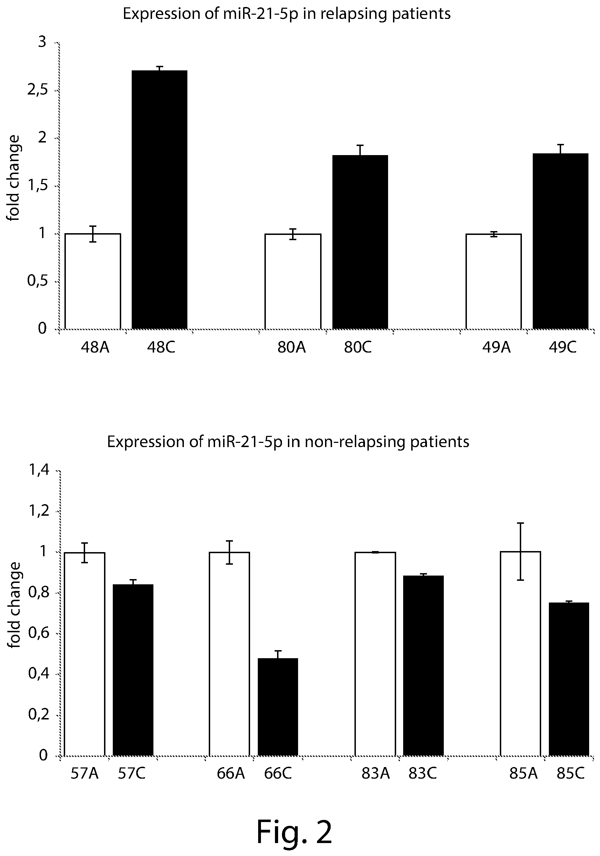

- **Figure 2** shows the expression of miR-21-5p normalised on the expression of spike-in cel miR-39 in 3 patients who had relapsed within 24 months after the surgical intervention and 4 patients who never relapsed and whose follow-up (FU) is at least 48 months long. In particular, sample A (preoperative) and sample C (15 days after the intervention) were analysed for each patient. As may be seen, in the case of patients who later relapsed, the expression of miR showed an increase in sample C compared to sample A, whereas in patients who had a good follow-up, the expression decreased; and

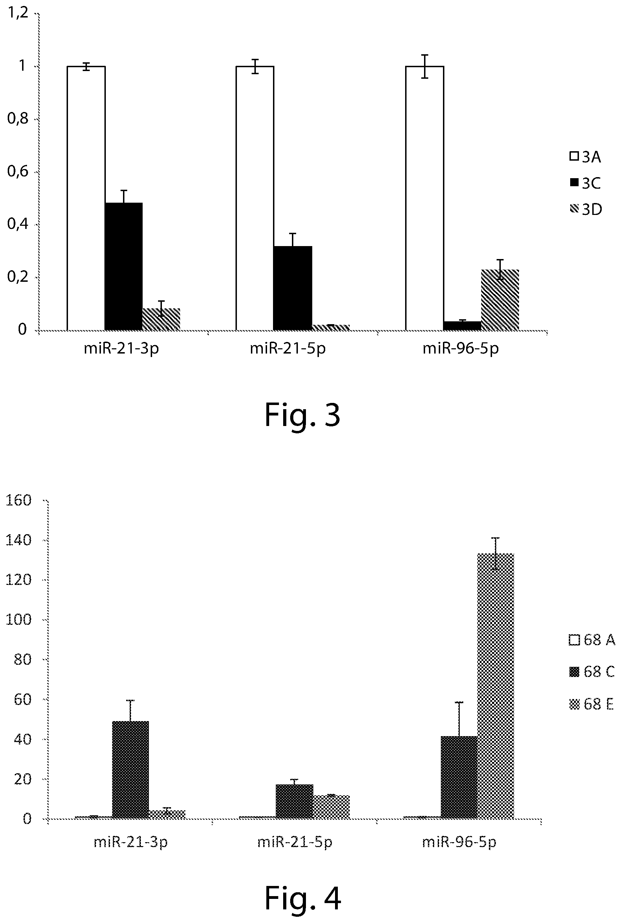

- **Figure 3** shows the evaluation of the expression of three microRNAs detectable in the serum of a non-relapsing patient with 60 months FU. The samples analysed were sample A (preoperative), sample C (15 days after the operation) and sample D (taken during one of the follow-up visits). Both in sample C and in sample D the expression of all three miRNAs showed a decrease compared to sample A.
- **Figure 4** shows the evaluation of the expression of three microRNAs detectable in the serum of a relapsing patient (recurrence developed about two years after the surgical intervention). The samples analysed were sample A (preoperative), sample C (15 days after the operation) and sample E (taken during one of the follow-up visits in which the development of recurrence was diagnosed). Both in sample C and in sample E the expression of all three miRNAs showed an increase compared to sample A.

### EXAMPLE 1: Study of circulating miRNAs according to the present invention as biomarkers in the early diagnosis of the development of recurrence in patients with head and neck cancers in liquid biopsies

### Materials and Methods

*The biological material of human origin used in this experimentation was obtained with the express, free and informed consent of the persons from whom said material was collected to the collection of the samples and use of the biological material, based on current legislation.*

### IRE HNSCC COHORT

The patients for this study were recruited at the Otolaryngology Head and Neck Surgery Department of the National Cancer Institute "Regina Elena" (IRE) in Rome. The IRE cohort has already been described in previous studies (Ganci et al., 2011; Ganci et al., 2013; Ganci et al., 2016; Ganci et al., 2017) and includes a collection of tumoral, peritumoral (sample taken at a distance >1 cm from the tumour) and normal (surgical resection margin) samples from patients with histologically proven, operable head and neck squamous cell carcinoma (oral cavity, oropharynx, hypopharynx, and larynx), who had not received any antitumour therapy prior to the surgery. Detailed demographic, epidemiological and clinicopathological information (HPV status, TNM staging, therapeutic regime after surgery and follow-up) were available for all of the patients. Information on the mutational status of TP53 was also available for about two thirds of the patients. As a consequence of the inclusion/exclusion criteria applied, the cohort mostly included HPV-negative patients. In fact, the only anatomical site etiologically associated with HPV viral infection is, at present, the oropharynx (cancer of the base of the tongue and tonsils) and, since the standard treatment for HPV-positive cases provides for chemo/radiotherapy without (or before) surgical resection, this subgroup is scantily represented in the cohort in question.

About 300 patients were recruited consecutively into this cohort and blood samples (whole blood, plasma and serum) were collected for the last 120 patients of the cohort and continue to be collected for new recruits and for follow-ups. The collection of saliva was also introduced; samples were available for 60 patients. The blood and saliva samples were taken a) before the surgical intervention; b) 1 day after the surgical intervention (blood only); c) 15 days after the surgical intervention; and d) during the follow-up, with the frequency of clinical examinations scheduled in the department in a uniform manner for all patients.

### Collection and preparation of biological samples

The blood and saliva samples were taken when the patient had fasted and were processed within one hour after collection. In particular, the blood samples were centrifuged at 3500 rpm (1500 g) for 20 minutes at 4°C in order to obtain the plasma and serum. Immediately afterwards, the plasma and serum were aliquoted and frozen at -80°C pending processing. The saliva sample, by contrast, was directly aliquoted and stored at -80°C.

### RNA extraction, reverse transcription of cDNA and analysis of miRNA expression

RNA was extracted from the biological fluids by means of the MagMAX total RNA kit (Thermo Fisher) using 100 microlitres of serum or saliva and following the indications of the protocol. Even though the kit does not provide a specific protocol for extracting RNA from saliva, it was demonstrated (in collaboration with the Thermo Fisher specialist) that the protocol developed for serum also works with saliva. The RNA was then reverse transcribed with the TaqMan^{™} Advanced miRNA cDNA Synthesis Kit according to the indications of the protocol.

The expression of the miRNAs was then analysed by qPCR (QuantStudio 5 Real-Time PCR Systems, Thermo Fisher) using TaqMan advanced chemistry and following the indications of the protocol.

The TaqMan assays used to analyse the expression of the miRNAs are the following:
Catalog number: A25576 (Thermo Fisher)
hsa-miR-429 (ID: 477849_mir)
hsa-miR-96-5p (ID: 478215_mir)
hsa-miR-21-5p (ID: 477975_mir)
hsa-miR-21-3p (ID: 477973_mir)

The analysis of expression of the sample was performed in triplicate (technical replicates). As an internal control and normaliser, use was made of two spike-ins (cel-miR-39 and cel-miR54) introduced at the time of extraction in the final concentration of 1 pmole. The range of threshold cycles (ct) in which all 4 miRNAs came out was from 25 to 36 ct.

### Data analysis

After having verified the quality of the raw data, such as the standard deviation calculated for the 3 technical replicates done for each sample and the level of fluorescence shown in the graph of the threshold cycles, the data were first normalised on the expression of the two spike-ins by means of the "delta delta Ct' (ΔΔCt)" method and then compared with sample A, which was taken as a reference in order to verify the modulation of the miRNAs in the subsequent samples. If the expression of one or more miRNAs is greater than or equal to sample A, the patient is considered to be at high risk of recurrence; therefore, stricter monitoring and a more aggressive treatment are recommended. If, by contrast, the expression of all of the miRNAs detected is lower than the baseline level, the patient is considered to be at low risk of recurrence. If, during the course of the FU, the levels of the miRNAs rise, it is highly probable that the tumour is reforming. In this case it is preferable to do further investigations and change the therapeutic strategy.

### Results

### Detection of the expression of the 4 miRNAs is feasible in the liquid biopsy samples of HNSCC.

The expression of the miRNAs miR-21-3p, miR-96-5p, miR-429 and miR-21-5p was tested in the liquid biopsy, in particular in saliva and blood samples (Fig.1).

The data obtained confirmed the feasibility of detection of the miRNAs in blood and saliva (Figure 1). In particular, miR-21-5p, miR-21-3p and the miR-96-5p were detectable in blood samples, whilst miR-429 was not present in any of the blood samples analysed. In saliva samples, by contrast, miR-21-5p, miR-21-3p and miR-429 were detectable, but not miR-96-5p.

Furthermore, in accordance with the literature (Zarhan et al., 2015; Hsu et al., 2012; Hou et al., 2015; Ishinaga et al., 2019), the serum expression data of miR-21-5p, one of the miRNAs that can be included among the ones making up the signature, confirm its role as a prognostic factor for the development of recurrence of HNSCC in liquid biopsies (Fig. 2).

Fig.3 shows the expression of the 3 miRNAs detectable in serum (miR-21-5p, miR-21-3p and miR-96-5p) in a non-relapsing patient with 60 months FU. The samples analysed are sample A (preoperative), sample C (15 days after the operation) and sample D (taken during one of the follow-up visits). As may be observed from the figure, both in sample C and in sample D the expression of all three miRNAs showed a decrease compared to sample A.

Figure 4 instead shows the pattern of expression of the miRNAs in a relapsing patient. In this case, the expression of all three miRNAs increased in samples C and E compared to sample A.

On the basis of the results obtained, it can be affirmed that the analysis of expression of the miRNAs miR-21-3p, miR-96-5p and miR-429 and, optionally, also of miR-21-5p, in both types of liquid biopsy sample, provides the possibility of correlating the expression of the miRNAs with the patient's clinical course. In particular, the presence of an increased or unchanged expression of at least one or more of the above-mentioned miRNAs in the liquid biopsy samples taken at times following the surgical removal of the tumour, compared to the expression thereof detected in the preoperative condition (thus in the presence of the tumour), is capable of indicating either a return of disease or residual disease.

The present invention has been described by way of non-limiting illustration according to preferred embodiments thereof.

### References

1.MiRNAs as biomarkers and therapeutic targets in cancer. HM Heneghan, N Miller, MJ Kerin - Current opinion in pharmacology, 2010.
2.miRNA expression profiles in head and neck squamous cell carcinoma and adjacent normal tissue. L Ramdas, U Giri, CL Ashorn... - Head & Neck, 2009.
3.Plasma MicroRNA as a novel diagnostic. Szelenberger R, Kacprzak M, Saluk-Bijak J, Zielinska M, Bijak M. Clin Chim Acta. 2019.
4.Circulating MicroRNAs in Cancer: Potential and Challenge. Cui M, Wang H, Yao X, Zhang D, Xie Y, Cui R, Zhang X, Front Genet. 2019.
5.Circulating miRNAs, Circulating Tumor Cells, and Cell-Free Circulating Tumor DNA in Head and Neck Cancer. Cheng-Ming Hsu, Chung-hui Ching, Chih-Yen Chien, Chung-Feng Hwang, Ming-Yu Yang, Ming-Shao Tsai, Yao-Te Tsai, Chun-Han Tseng, International Journal of Head and Neck Science, 2018.
6.Salivary MicroRNAs for Early Detection of Head and Neck Squamous Cell Carcinoma: A Case-Control Study in the High Altitude Mestizo Ecuadorian Population. Carolina Salazar-Ruales, Jessica-Viviana Arguello, Andrés López-Cortés, Alejandro Cabrera-Andrade, Jennyfer M. García-Cárdenas, Patricia Guevara-Ramírez, Patricio Peralta, Paola E. Leone, and César Paz-y-Miño. Biomed Res Int. 2018
7.Human salivary microRNAs in Cancer. Oscar Rapado-González, Blanca Majem, Laura Muinelo-Romay, Ana Álvarez-Castro, Anna Santamaría, Antonio Gil-Moreno, Rafael López-López, and Maria Mercedes Suárez-Cunqueiro1, J Cancer. 2018.
8.Salivary microRNAs in oral cancer. Zahran F, Ghalwash D, Shaker O. et al.. Oral Dis. 2015.
9.Circulating miRNA is a novel marker for head and neck squamous cell carcinoma. Hsu CM, Lin PM, Wang YM, Chen ZJ, Lin SF, Yang MY. Tumour Biol. 2012
10.Circulating microRNAs as novel prognosis biomarkers for head and neck squamous cell carcinoma. Hou B, Ishinaga H, Midorikawa K, Shah SA, Nakamura S, Hiraku Y, Oikawa S, Murata M, Takeuchi K. Cancer Biol Ther. 2015.
11.A longitudinal study on circulating miR-21 as a therapeutic effect marker in head and neck squamous cell carcinoma. Ishinaga H, He F1, Hou B, Shah S, Murata M, Takeuchi K. Carcinogenesis. 2019.
12.A panel of serum exosomal microRNAs as predictive markers for chemoresistance in advanced colorectal cancer. Jin G, Liu Y, Zhang J, Bian Z, Yao S, Fei B, Zhou L, Yin Y, Huang Z. Cancer Chemother Pharmacol. 2019
13.Tumor microRNA expression profiling identifies circulating microRNAs for early breast cancer detection. Matamala N, Vargas MT, González-Cámpora R, Miñambres R, Arias JI, Menéndez P, Andrés-León E, Gómez-López G, Yanowsky K, Calvete-Candenas J, Inglada-Pérez L, Martinez-Delgado B, Benítez J. Clin Chem. 2015.
14.Plasma MicroRNA Pair Panels as Novel Biomarkers for Detection of Early Stage Breast Cancer. Fang R, Zhu Y, Hu L, Khadka VS, Ai J, Zou H, Ju D, Jiang B, Deng Y, Hu X. Front Physiol. 2019.
15.Allelic expression imbalance of TP53 mutated and polymorphic alleles in head and neck tumors. Ganci F, Conti S, Fontemaggi G, Manciocco V, Donzelli S, Covello R, Muti P, Strano S, Blandino G, Spriano G. OMICS. 2011
16.Expression of TP53 mutation-associated microRNAs predicts clinical outcome in head and neck squamous cell carcinoma patients. Ganci F, Sacconi A, Bossel Ben-Moshe N, Manciocco V, Sperduti I, Strigari L, Covello R, Benevolo M, Pescarmona E, Domany E, Muti P, Strano S, Spriano G, Fontemaggi G, Blandino G. Ann Oncol. 2013
17.MicroRNA expression as predictor of local recurrence risk in oral squamous cell carcinoma. Ganci F, Sacconi A, Manciocco V, Sperduti I, Battaglia P, Covello R, Muti P, Strano S, Spriano G, Fontemaggi G, Blandino G. Head Neck. 2016.
18. Altered peritumoral microRNA expression predicts head and neck cancer patients with a high risk of recurrence. Ganci F, Sacconi A, Manciocco V, Covello R, Benevolo M, Rollo F, Strano S, Valsoni S, Bicciato S, Spriano G, Muti P, Fontemaggi G, Blandino G. Mod Pathol. 2017
19. Comparison of Prognostic MicroRNA Biomarkers in Blood and Tissues for Gastric Cancer. Wenying Yan, Laijun Qian, Jiajia Chen, Weichang Chen, and Bairong Shen. J Cancer. 2016; 7(1): 95-106.doi: 10.7150/jca.13340
20. MicroRNA-based classifiers for diagnosis of oral cavity squamous cell carcinoma in tissue and plasma. Nicklas Juel Pedersen, David Hebbelstrup Jensen, Giedrius Lelkaitis, Katalin Kiss, Birgitte Wittenborg Charabi, Henrik Ullum, Lena Specht, Ane Yde Schmidt, Finn Cilius Nielsen, Christian von Buchwald. Oral Oncol 2018 Aug;83:46-52. doi: 10.1016/j.oraloncology.2018.05.020
21. Distinct spectrum of microRNA expression in forensically relevant body fluids and probabilistic discriminant approach. Shuntaro Fujimoto, Sho Manabe, Chie Morimoto, Munetaka Ozeki , Yuya Hamano, Eriko Hirai, Hirokazu Kotani, Keiji Tamaki. Sci Rep. 2019 Oct 4;9(1):14332. doi: 10.1038/s41598-019-50796-8.
22. Detection of somatic mutations and HPV in the saliva and plasma of patients with head and neck squamous cell carcinomas. Yuxuan Wang, Simeon Springer, Carolyn L Mulvey, Natalie Silliman, Joy Schaefer, Mark Sausen, Nathan James, Eleni M Rettig, Theresa Guo, Curtis R Pickering , Justin A Bishop, Christine H Chung , Joseph A Califano, David W Eisele, Carole Fakhry, Christine G Gourin, Patrick K Ha, Hyunseok Kang , Ana Kiess , Wayne M Koch, Jeffrey N Myers, Harry Quon, Jeremy D Richmon, David Sidransky, Ralph P Tufano, William H Westra, Chetan Bettegowda, Luis A Diaz Jr, Nickolas Papaafterulos, Kenneth W Kinzler , Bert Vogelstein, Nishant Agrawal. Sci Transl Med. 2015 Jun 24;7(293):293ra104. doi: 10.1126/scitranslmed.aaa8507.
23. miR-96-5p targets PTEN expression affecting radiochemosensitivity of HNSCC cells. Mahrou Vahabi, Claudio Pulito, Andrea Sacconi, Sara Donzelli, Marco D'Andrea, Valentina Manciocco, Raul Pellini, Paola Paci, Giuseppe Sanguineti, Lidia Strigari, Giuseppe Spriano, Paola Muti, Pier Paolo Pandolfi, Sabrina Strano, Shahrokh Safarian, Federica Ganci , Giovanni Blandino. J Exp Clin Cancer Res. 2019 Mar 29;38(1):141. doi: 10.1186/s13046-019-1119-x.

## Claims

1. Method for *in vitro* diagnosis of the risk of recurrence or persistence of head and neck cancer in a patient who underwent removal of the tumour, said method comprising or consisting of measuring, in at least one liquid biopsy sample collected before tumour removal and in at least one liquid biopsy sample collected after tumour removal, the expression
of two or all of the miRNAs selected from miR-21-3p, miR-96-5p and miR-429, optionally also of the microRNA miR-21-5p; or
of one of the miRNAs selected from miR-21-3p, miR-96-5p and miR-429 and also of the microRNA miR-21-5p,
wherein an expression of said miRNAs in said at least one liquid biopsy sample collected after tumour removal that is equal to or greater than the expression of said miRNAs in said at least one liquid biopsy sample collected before tumour removal indicates a high risk of recurrence or persistence of the cancer, whereas a lower expression indicates a low risk of recurrence or persistence of the cancer.

2. Method according to claim 1, wherein said at least one liquid biopsy sample is selected from blood, plasma, serum and saliva.

3. Method according to any one of claims 1-2, wherein the expression of miR-96-5p is measured in a blood, serum or plasma sample, the expression of miR-429 is measured in a saliva sample and the expression of miR-21-3p or miR-21-5p is measured in a blood, plasma, serum or saliva sample.

4. Method according to any one of claims 1-3, wherein said method is carried out on one or more samples selected from blood, serum or plasma and on a saliva sample.

5. Method according to any one of claims 1-4, wherein said two miRNAs or said one of the miRNAs in combination with miR-21-5p comprise miR-96-5p when the patient undergoes radio- and/or chemotherapy, said miR-96-5p being preferably detected in a sample selected from serum, blood or plasma.

6. Method according to any one of claims 1-5, wherein said method is carried out by Real Time PCR, Microarray or RNA Next Generation Sequencing.

7. Use of two or all of the microRNAs selected from miR-21-3p, miR-96-5p and miR-429, optionally in combination with the microRNA miR-21-5p; or
of one of the miRNAs selected from miR-21-3p, miR-96-5p and miR-429 in combination with the microRNA miR-21-5p, as biomarkers for *in vitro* diagnosis of the degree of risk of recurrence or persistence of head and neck cancer in liquid biopsy samples.

8. Use according to claim 7, wherein the liquid biopsy samples are selected from blood, plasma, serum and/or saliva.

## Patentansprüche

1. Verfahren zur *In-vitro*-Diagnose des Risikos eines Rezidivs oder Fortbestehens von Kopf-Hals-Krebs bei einem Patienten, bei dem der Tumor entfernt wurde, wobei das Verfahren das Messen der Expression in mindestens einer vor der Tumorentfernung entnommenen Flüssigbiopsieprobe und in mindestens einer nach der Tumorentfernung entnommenen Flüssigbiopsieprobe umfasst oder daraus besteht
von zwei oder allen der aus miR-21-3p, miR-96-5p und miR-429 ausgewählten miRNAs, gegebenenfalls auch der microRNA miR-21-5p; oder
von einer der aus miR-21-3p, miR-96-5p und miR-429 ausgewählten miRNAs und zusätzlich der microRNA miR-21-5p,
wobei eine Expression der genannten miRNAs in der genannten mindestens einen nach der Tumorentfernung entnommenen Flüssigbiopsieprobe, die gleich der oder größer als die Expression der genannten miRNAs in der genannten mindestens einen vor der Tumorentfernung entnommenen Flüssigbiopsieprobe ist, ein hohes Risiko eines Rezidivs oder Fortbestehens des Krebses anzeigt, während eine niedrigere Expression ein geringes Risiko eines Rezidivs oder Fortbestehens des Krebses anzeigt.

2. Verfahren nach Anspruch 1, wobei die genannte mindestens eine Flüssigbiopsieprobe aus Blut, Plasma, Serum und Speichel ausgewählt ist.

3. Verfahren nach einem der Ansprüche 1-2, wobei die Expression von miR-96-5p in einer Blut-, Serum- oder Plasmaprobe gemessen wird, die Expression von miR-429 in einer Speichelprobe gemessen wird und die Expression von miR-21-3p oder miR-21-5p in einer Blut-, Plasma-, Serum- oder Speichelprobe gemessen wird.

4. Verfahren nach einem der Ansprüche 1-3, wobei das genannte Verfahren an einer oder mehreren aus Blut, Serum oder Plasma ausgewählten Proben und an einer Speichelprobe durchgeführt wird.

5. Verfahren nach einem der Ansprüche 1-4, wobei die genannten zwei miRNAs oder die genannte eine der miRNAs in Kombination mit miR-21-5p miR-96-5p umfassen, wenn sich der Patient einer Strahlen- und/oder Chemotherapie unterzieht, wobei die genannte miR-96-5p vorzugsweise in einer aus Serum, Blut oder Plasma ausgewählten Probe nachgewiesen wird.

6. Verfahren nach einem der Ansprüche 1-5, wobei das genannte Verfahren mittels Real Time PCR, Microarray oder RNA Next Generation Sequencing durchgeführt wird.

7. Verwendung von zwei oder allen der aus miR-21-3p, miR-96-5p und miR-429 ausgewählten microRNAs, gegebenenfalls in Kombination mit der microRNA miR-21-5p; oder
von einer der aus miR-21-3p, miR-96-5p und miR-429 ausgewählten miRNAs in Kombination mit der microRNA miR-21-5p als Biomarker für die *In-vitro*-Diagnose des Ausmaßes des Risikos eines Rezidivs oder Fortbestehens von Kopf-Hals-Krebs in Flüssigbiopsieproben.

8. Verwendung nach Anspruch 7, wobei die Flüssigbiopsieproben aus Blut, Plasma, Serum und/oder Speichel ausgewählt sind.

## Revendications

1. Procédé de diagnostic *in vitro* du risque de récidive ou de persistance d'un cancer de la tête et du cou chez un patient ayant subi une ablation de la tumeur, ledit procédé comprenant ou consistant à mesurer, dans au moins un échantillon de biopsie liquide prélevé avant l'ablation de la tumeur et dans au moins un échantillon de biopsie liquide prélevé après l'ablation de la tumeur, l'expression
de deux ou de la totalité des miRNA choisis parmi miR-21-3p, miR-96-5p et miR-429, éventuellement également du microRNA miR-21-5p; ou
de l'un des miRNA choisis parmi miR-21-3p, miR-96-5p et miR-429 et également du microRNA miR-21-5p,
dans lequel une expression desdits miRNA dans ledit au moins un échantillon de biopsie liquide prélevé après l'ablation de la tumeur qui est supérieure ou égale à l'expression desdits miRNA dans ledit au moins un échantillon de biopsie liquide prélevé avant l'ablation de la tumeur indique un risque élevé de récidive ou de persistance du cancer, tandis qu'une expression inférieure indique un faible risque de récidive ou de persistance du cancer.

2. Procédé selon la revendication 1, dans lequel ledit au moins un échantillon de biopsie liquide est choisi parmi le sang, le plasma, le sérum et la salive.

3. Procédé selon l'une quelconque des revendications 1-2, dans lequel l'expression de miR-96-5p est mesurée dans un échantillon de sang, de sérum ou de plasma, l'expression de miR-429 est mesurée dans un échantillon de salive et l'expression de miR-21-3p ou de miR-21-5p est mesurée dans un échantillon de sang, de plasma, de sérum ou de salive.

4. Procédé selon l'une quelconque des revendications 1-3, dans lequel ledit procédé est mis en œuvre sur un ou plusieurs échantillons choisis parmi le sang, le sérum ou le plasma, ainsi que sur un échantillon de salive.

5. Procédé selon l'une quelconque des revendications 1-4, dans lequel lesdits deux miRNA ou l'un des miRNA en combinaison avec miR-21-5p comprennent miR-96-5p lorsque le patient reçoit une radio- et/ou une chimiothérapie, ledit miR-96-5p étant de préférence détecté dans un échantillon choisi parmi le sérum, le sang ou le plasma.

6. Procédé selon l'une quelconque des revendications 1-5, dans lequel ledit procédé est mis en œuvre par PCR en temps réel, par microréseau ou par séquençage de nouvelle génération de l'RNA.

7. Utilisation de deux ou de la totalité des microRNA choisis parmi miR-21-3p, miR-96-5p et miR-429, éventuellement en combinaison avec le microRNA miR-21-5p; ou
de l'un des miRNA choisis parmi miR-21-3p, miR-96-5p et miR-429 en combinaison avec le microRNA miR-21-5p, en tant que biomarqueurs pour le diagnostic *in vitro* du degré de risque de récidive ou de persistance d'un cancer de la tête et du cou dans des échantillons de biopsie liquide.

8. Utilisation selon la revendication 7, dans laquelle les échantillons de biopsie liquide sont choisis parmi le sang, le plasma, le sérum et/ou la salive.
